(19) Europäisches Patentamt
European Patent Office
Office européen des brevets

(11) Publication number: **0 271 607 B1**

# (12) EUROPEAN PATENT SPECIFICATION

(45) Date of publication of patent specification: **18.09.91** (51) Int. Cl.[5]: **A23K 1/16**

(21) Application number: **86202281.1**

(22) Date of filing: **16.12.86**

## (54) 2-Pyridinol compositions and methods of use as anabolic agents.

(43) Date of publication of application:
**22.06.88 Bulletin 88/25**

(45) Publication of the grant of the patent:
**18.09.91 Bulletin 91/38**

(84) Designated Contracting States:
**BE DE FR GB IT NL**

(56) References cited:
**EP-A- 0 102 184**
**US-A- 3 206 358**
**US-A- 4 474 789**

(73) Proprietor: **THE DOW CHEMICAL COMPANY**
**2030 Dow Center Abbott Road P.O. Box 1967**
**Midland Michigan 48640-1967(US)**

(72) Inventor: **Ware, Douglas Robert**
**3104 Noeske Street**
**Midland Michigan 48640(US)**
Inventor: **Hymas, Theo Alfred**
**4500 Linden**
**Midland Michigan 48640(US)**

(74) Representative: **Smulders, Theodorus A.H.J., Ir. et al**
**Vereenigde Octrooibureaux Nieuwe Parklaan 107**
**NL-2587 BP 's-Gravenhage(NL)**

**EP 0 271 607 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method of producing an anabolic response in animals by administering to the animals an anabolic agent whereby such response is exhibited in one or more of the following ways:

(a) an increased growth rate
(b) increased milk production
(c) increased fiber production
(d) increased protein content,
(e) improved reproductive performance and
(f) increased feed conversion efficiency in animals.

In EP-A 102,184 the use of copper salts of 2-hydroxipyridine-N-oxides as active ingredients in feed compositions for increasing growth and feed efficiency of animals has been disclosed.

US-A 4,474,789 is directed to the use of (trichloromethyl)pyridine compounds for improving growth rate and feed efficiency in ruminants.

More particularly, the present invention relates to a method of obtaining the above anabolic responses by administering to an animal an anabolically effective amount of a substituted-2-pyridinol or a physiologically accetable salt or ester thereof.

Briefly, in accordance with the present invention, the anabolic response in animals, for example, the feed conversion efficiency, milk and fiber production, protein content, reproductive performance, and growth rate of meat-bearing animals, can be increased by administering to said animals an anabolically effective amount of a compound corresponding to the following formula

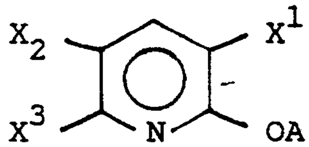

(I)

wherein

$X^1$, $X^2$ and $X^3$ independently represent halogen, preferably chloro;

A represents hydrogen, alkali metal, alkaline earth metal, zinc, iron, manganese or

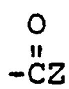 ; and

Z represents $C_1$-$C_{11}$ alkyl, $C_1$-$C_{11}$ haloalkyl or $C_1$-$C_{11}$ hydroxyalkyl.

Preferably, $X^1$, $X^2$ and $X^3$ are chlorine. Also preferred is that A is an alkali metal, more preferably sodium.

For convenience, the compounds of Formula I will be hereinafter referred to as the "pyridinol compounds".

When used herein, the term "alkyl" is meant to encompass straight and branched chain alkyl groups and cycloalkyl groups of from 3 carbon atoms up to the upper carbon atom limits as specifically set forth. The term "$C_1$-$C_{11}$" indicates that from one to 11 carbon atoms may be present in a group.

It is well known that pyridinols exist in the pyridinol and tautomeric pyridone form. When any reference is made to present pyridinol compounds of Formula I where A is hydrogen, it is understood to encompass both tautomeric forms.

The term "anabolic response" refers to the effects exhibited by an animal in response to an anabolic agent. When an anabolic agent is administered to an animal the anabolic response is exhibited by, for example, an increase in weight gain, increase in fiber production, increase in milk production in lactating animals, increase in feed conversion efficiency, increase in protein content or improved reproductive performance when compared to animals not receiving the anabolic agent.

An important objective of this invention is to produce an anabolic response in animals which is manifested by, for example, one or more of the following: increased weight gain, increased protein content, increased wool production, improved reproductive performance, increased milk production in lactating animals, and increased feed conversion efficiency. Examples of animals which exhibit increased growth rate

when administered an anabolically effective amount of one or more of the present pyridinol compounds are commercial meat-bearing animals such as cattle, sheep, swine, and poultry.

In commercial practice, immature sheep, cattle and swine are commonly fed for maximum growth rate in feed lots and poultry, such as chickens and turkeys, are raised in broiler pens, until they reach a marketable weight. When the desired weight of the animals is achieved, they are then sold for slaughter. It is important economically, that the animals achieve market weight in as short a time as possible, while consuming the least amount of food necessary to achieve such gain. It has been unexpectedly found that when anabolically effective levels of one or more of the present pyridinol compounds are administered to animals of the classes described hereinabove, they gain weight at a faster rate while consuming less feed per pound of gain resulting in better overall economic efficiency, and reflecting the fact that administration of the present pyridinol compounds results in an anabolic response.

The present pyridinol compounds can be administered to animals orally by dosage forms, such as, in admixture with food, and additionally in the form of boluses, capsules, tablets, suspensions or solutions containing the present pyridinol compounds. The compounds can also be administered parenterally, such as, for example, intramuscularly or intravenously, or by way of an implant which slowly releases the pyridinol compound into the tissue or blood stream of the animal.

The anabolically effective amount of the present pyridinol compounds exists as a range of from 0.01 to 20 milligrams per kilogram (kg) of body weight of the animal per day. This effective range can vary depending on the size of the animal, the species of the animal, the age of the animal, the type of feed used, the active compound used, or the route of administration of the active compound. The optimum range of an effective amount, based on the above-mentioned variables, can be found using conventionally known techniques, i.e., dose titration determinations.

An anabolically effective amount of the present pyridinols can be conveniently administered substantially daily for at least 7 days, preferably at least 28, more preferably at least 90 days and even more preferably throughout the life of the animal.

Lactating animals exhibit an increase in milk production when administered an anabolically effective amount of the present pyridinols. Lactating animals seem to be more sensitive to the effects of the present pyridinol compounds and thus may require a lower dose of the present pyridinol compounds to produce an increase in milk production when compared to an anabolically effective dose in other animals to produce an increase in body weight gain.

Animals raised for their fiber production, such as sheep, exhibit an increase in fiber production when administered an anabolically effective amount of the present pyridinol compounds.

The present pyridinol compounds are conveniently incorporated in a feed composition in an appropriate amount to achieve the desired daily dosage in the amount of ration or supplement consumed regularly. For example, one or more of the present pyridinol compounds are conveniently incorporated in a feed composition from generally 0.5 to 1600 grams per ton [metric] of complete ration depending on the age and type of animal. The term "ton", "tonne" or "ton [metric]" is intended to mean, in this specification, a metric ton of 1000 kilograms. The "ton" in parenthesis associated with "lb" or "pounds" units is intended to mean the short ton of 907 kilograms. The present pyridinol compounds may also be incorporated in a mineral, protein or energy-type feed additive supplement in an appropriate amount to provide anabolically effective daily dosages.

For commercial use, it is convenient to provide a feed additive premix or concenrate containing one or more of the present pyridinol compounds in a proportion such that a predetermined quantity of the premix is to be added per ton [metric] of complete ration, for example, from 0.2 to 454 kg (0.5 to 1,000 pounds) contains from 0.5 to 1600 grams of the present pyridinol compounds. The feed additive premix or concentrate comprises one or more of the present pyridinol compounds and a carrier such as soybean meal or ground corn or other edible feed grade material or innocuous diluent, such as, alcohols, glycols or molasses, suitable for the animal at hand. A concentrate may contain from 2 to 98 percent by weight of one or more of the present pyridinol compounds in intimate admixture with an adjuvant therefor.

The present pyridinol compounds, when administered parenterally, are preferably dissolved in sterile distilled water or other physiologically acceptable liquid media and compounded in accordance with the known pharmaceutical art.

The term "feed conversion efficiency" refers to the total amount of feed consumed over a period of time divided by the amount of body weight gain over that period as seen in the following formula:

$$\frac{\text{feed consumed over time period}}{\text{gain in body weight over time period}}$$

The term "increased feed conversion efficiency" refers to a more efficient means of bringing animals to market weight. An increase in feed conversion efficiency will be reflected by a lower numerical value of the feed conversion efficiency number when compared to a lower feed conversion efficiency.

The term "increased protein content", when referred to as being an anabolic response, means carcass alteration of an animal exhibited by a relative increase in body protein content and a relative decrease in body fat content.

One of the practical effects of this invention is to bring animals such as sheep, cattle, swine, and poultry promptly to market weight with minimal feed consumption. The present pyridinol compounds are most conveniently dispersed uniformly throughout the normal feed or feed additive supplement of the subject animal in anabolically effective dosage levels. For example, an anabolically effective amount of the present pyridinol compounds is supplied in an animal feed comprising from 0.0005 to 16 percent by weight of one or more of the present pyridinol compounds and more preferably from 0.005 to 0.10 percent by weight corresponding to 50 and 100 parts per million (ppm). Such animal feeds should, when fed to an animal, provide to the animal from 0.01 to 20 milligrams per kilogram of body weight per day of one or more of the present pyridinol compounds.

In further embodiments of the method of the present invention, compositions containing the present pyridinol compounds can be advantageously employed in combination with one or more additional feed additives such as coccidiostats, antibiotics, minerals, vitamins or the like.

The animal feeds most generally used in conjunction with this invention are composed of various grains and/or grain mixtures and/or roughage feeds such as hay, cotton seed hulls, rice hulls, silage, or other high fiber feedstuffs commonly fed to meat-, milk-, and/or wool-producing animals, especially in cattle or sheep feeds. The feeds for swine and poultry will consist primarily of various grain mixtures plus the usual additaments such as bran meal, soybean meal, cotton seed meal, tankage or alfalfa meals suitable for monogastric animals.

Examples of carriers for premix or concentrate compositions are soybean meal, corn oil, ground corn, ground corn cobs, barley, wheat, mineral mixtures containing, e.g., vermiculite or diatomaceous earth, corn gluten meal, corn distillers' solubles, soy flour or other modestly priced edible ingredients. The active ingredient will be in amounts to satisfy the criteria set forth above for balanced feed rations. This premix or concentrate is then in turn mixed uniformly with the normal diet for the animal as desired by the grower or the feed mixer. The above mentioned grains, grain mixtures, roughage feeds, usual additaments, carriers and innocuous diluents constitute acceptable adjuvants for purposes of this invention.

As indicated hereinabove, the amount of the present pyridinol compounds added to such feeds will be in the range of from 0.5 to 1600 grams per ton [metric] of feed (dry matter basis), depending on the age and type of animal. More preferably the amount added to such feeds will be in the range of from 5 to 700 grams per ton [metric]. Very young animals that have been weaned or young poultry one or a few days old, will have a lower feed consumption compared to an older animal. However, as the animal goes through a growth period to a fattening period, sometimes called finishing, the feed consumption gradually increases, but generally falls in proportion to body weight.

The daily dosage of a preferred compound, 3,5,6-trichloro-2-pyridinol or a physiologically acceptable salt thereof, in swine falls in the range of from 0.01 to 20 milligrams per kilogram of body weight. More preferably, in the range of from 0.1 to 10 milligrams per kilogram of body weight, and even more preferably from 2.5 to 5 milligrams per kilogram of body weight. Examples of physiologically acceptable salts of 3,5,6-trichloro-2-pyridinol are the Na, K, Ca, Mg, Mn, Zn and iron salts thereof.

Based on known information concerning the average feed intake for various sizes and types of animals, the following amounts of one or a mixture of two or more of the present pyridinol compounds are incorporated into each ton of animal feed in order to provide an anabolically effective dose described herein. For example, cattle on a growing diet will ordinarily be fed a diet containing from 3 to 800 grams of one or a mixture of the present pyridinol compounds per ton of feed on a dry matter basis (DM), while cattle on a fattening diet will be fed a feed containing from 3 to 1000 grams of one or a mixture of the present pyridinol compounds per ton [metric] (DM) (0.007 to 2.0 lb/ton). Maintenance diets fed lactating dairy cattle should contain from 5 to 1600 grams of one or a mixture of the present pyridinol compounds per ton [metric] of feed (DM) (.01 to 3.60 lbs/ton), depending on the size and feed intake of the animal. Non-lactating dairy cattle should receive a feed containing 5 to 1000 grams of one or a mixture of the present pyridinol compounds per ton of feed (DM) (0.01 to 2.70 lbs/ton). Lambs on dry feed will generally be fed a ration containing 2 to 600 grams of one or a mixture of the present pyridinol compounds per ton of feed (DM) (0.004 to 1.4 lbs/ton). Grower pigs may be fed a ration containing 1 to 400 grams of one or a mixture of the present pyridinol compounds per ton of feed (DM) while swine in the fattening stage will generally be supplied a ration containing 2 to 500 grams per ton (DM) (0.002 to 1.20 lbs/ton). Poultry such as very small day-old or older birds up through starter or grower stage will generally be fed a complete ration of mash containing 0.5 to 400 grams of one or a mixture of the present pyridinol compounds per ton of feed (DM) while poultry on a fattening diet will feed on a ration containing 1 to 400 grams per ton (DM) (0.001 to 0.8 lb/ton).

The following examples further illustrate the practice of the present invention.

Example 1:

A typical growing ration for ruminants is as follows:

| Ingredients | Weight Percent (D.M. Basis) |
|---|---|
| Mixed Hay | 40.0 |
| Ground Yellow Corn | 45.0 |
| Soybean Oil Meal | 7.0 |
| Can Molasses | 7.0 |
| Dicalcium Phosphate | 0.5 |
| Trace Mineral Salt | 0.5 |
| | 100.0 |

In addition to the above the following supplements are added.

| | |
|---|---|
| Vitamin A | 396µg/kg (300 IU/lb) |
| Vitamin D | 330µg/kg (150 IU/lb) |
| 3,5,6-Trichloro-2-pyridinol or a physiologically acceptable salt thereof | 3.5 to 450 grams/ton of feed |

Such a feed typically contains 8 to 15 percent by weight moisture.

Example 2:

A typical finishing ration for ruminants is as follows:

| Ingredients | Weight Percent (D.M. Basis) |
|---|---|
| Ground Shelled Corn | 65.85 |
| Mixed Ground Hay | 20.00 |
| Dried Molasses | 6.00 |
| Soybean Meal | 6.00 |
| Trace Mineral Salt | 0.50 |
| Dicalcium Phosphate | 0.40 |
| Ground Limestone | 0.70 |
| | 99.45 |

In addition to the above the following supplements are added.

| | |
|---|---|
| Vitamin A (300,000 units/gms) | 66.7 grams/ton |
| Vitamin $D_2$ (16,000,000 units/lb) | 7.1 grams/ton |
| 3,5,6-Trichloro-2-pyridinol or a physiologically acceptable salt thereof | 3.5 to 450 grams/ton of feed |

Such a feed typically contains 8 to 15 percent by weight moisture.

Example 3

An example of a suitable feed additive premix is as follows:

| | |
|---|---|
| 3,5,6-Trichloro-2-pyridinol or a physiologically acceptable salt thereof | 64 grams |
| Ground Yellow Corn (5-10% moisture) | 390 grams |

Example 4

For use in the field for animals on range, the present pyridinol compounds may be administered by means of salt or molasses blocks. A typical block is prepared using the following compositions:

| Ingredients | Weight Percent (D.M. Basis) |
|---|---|
| Dried Cane Molasses | 35.00 |
| Ground Soybean Hulls | 29.60 |
| 3,5,6-Trichloro-2-pyridinol or a physiologically acceptable salt thereof* | 5.36 |
| Granulated Salt | 25.90 |
| Trace Minerals and Vitamins | 0.24 |
| Stabilized Animal Fat | 1.30 |
| Moisture | 2.60 |
| | 100.00 |

*Provided that the field animal ingests enough of the block per day to provide an effective dose of active ingredient hereinbefore mentioned.

Example 5

If desired, the present pyridinol compounds may be administered as a part of a liquid animal feed supplement such as a supplement containing a non-protein nitrogen source such as urea or ammonium sulfate in admixture with molasses and other feed ingredients. Such a liquid supplement is prepared using the following formula:

| Ingredients | Weight Percent (D.M. Basis) |
|---|---|
| Molasses | 80.00 |
| Water | 16.25 |
| Urea or Ammonium Sulfate | 2.00 |
| Trace Minerals | .50 |
| Vitamin A, D & E | .05 |
| Salt | 1.00 |
| 3,5,6-Trichloro-2-pyridinol or a physiologically acceptable salt thereof* | .20 |
| | 100.00% |

*Provided the animal drinks enough of the liquid per day to provide an effective dose of active ingredient hereinbefore mentioned.

The following examples demonstrate the anabolic effects of the present pyridinols as seen by an increase in the average daily gain in weight, increased feed conversion efficiency, or increased milk production in feeding trials conducted in Europe and in the United States.

I. Protocol and Results for European Trials

The process of the present invention was tested in pig feed trials in 4 European countries as a pig growth and as a feed efficiency promoting agent on a total of 520 pigs, including controls. The countries included Denmark, England, Germany and Holland (Netherlands).

The use 2,3,5-trichloropyridinol (TriCP) in feed was from weaning to the end of the fattening period. A starter diet was fed to the pigs from the beginning of the weaning period, which was 25 kg (in Germany, 30 kg) to a weight of 50 kg (in Gemrnay, 65 kg). A finisher diet was fed to the pigs from a weight of 50 kg (in Germany, 65 kg) to the end of the fattening period which was 100 kg (in Germany, 105 kg). Dosage rates of zero "0" (the control), 50, 75 and 100 ppm of TriCP in the feed were tested. Protein supply and energy content of the starter and finisher diets was adapted to represent the most common nutritional balance in each country, as represented in Table 1.

Table 1

| Nutritional Data of Feed Used in European Trials | | | | | |
|---|---|---|---|---|---|
| Country | Number of Pigs | Diet | % Protein By Weight | Energy In Feed (KCal/Kg Feed) | % Lysine in Feed by Weight |
| Denmark | 128 | Starter<br>Finisher | 21.4<br>16.6 | 2098<br>2105 | 0.81<br>0.55 |
| England | 64 | Starter<br>Finisher | 17.0<br>15.7 | 2108<br>2150 | 0.89<br>0.77 |
| Germany | 168 | Starter<br>Finisher | 16.5<br>15.0 | 2210<br>2225 | 0.90<br>0.70 |
| Holland | 160 | Starter<br>Finisher | 17.0<br>13.6 | 2190<br>2185 | 0.96<br>0.70 |

Individual protocols for each European country are provided hereinafter.

IA. Denmark Trial

The Denmark trialplan is shown below.

| Feeding Trial Plan in Denmark | | | | |
|---|---|---|---|---|
| | Group Number | | | |
| | 1 | 2 | 3 | 4 |
| Number of Pigs | 32 | 32 | 32 | 32 |
| TriCP concentration in feed in ppm | 0 | 50 | 75 | 100 |

In each of the four groups there were 32 pigs, divided into 4 pens with 4 males and 4 pens with 4 female pigs. The trial was repeated 4 times. One repetition consisted of 10 pens, with the pigs derived mainly from the same livestock and the weight of the individual pigs differed as little as possible. All 4 groups were fed the same feed mixture; except that, groups 2, 3 and 4 received 50, 75 and 100 ppm TriCP, respectively.

As agreed with the Ministry of Agriculture and the Veterinary Directorate, a withdrawal period of 10 days after the latest supply of TriCP was followed. To do this all pigs in every repetition received the same feed mixture without additive as soon as the first pig had reached the time of 10 days before slaughter.

The following quantity of feed was given daily :

| Weight, kg | 20 | 30 | 40 | 50 | 60 | 70 | 80 |
|---|---|---|---|---|---|---|---|
| Feed units* per pig daily | 0.9 | 1.5 | 1.9 | 2.2 | 2.5 | 2.7 | 2.8 |

* Feed units are calculated according to the Danish Standard. In the feed composition used here it is equal to 1.010 kg.

Table 2 shows the composition and contents of the feed mixture used. The feed was in the form of meal and a new lot was produced every month. To the basic feed the TriCP additives were added. The table shows that up to 50 kg there was a percentage of 24 percent soya in the mixture and after 50 kg this was 12 percent.

Table 2

Feed Mixture Composition and Contents in Denmark Trial

| Period, kg | Up to 50 kg | After 50 kg |
|---|---|---|
| Barley % | 73.4 | 85.4 |
| Soya % | 24.0 | 12.0 |
| Chalk % | 0.8 | 0.8 |
| Dicalciumphosphate % | 1.2 | 1.2 |
| Salt % | 0.4 | 0.4 |
| Vitamine and microelements % * | 0.2 | 0.2 |
| Dry stuff % | 86.8 | 87.2 |
| **In % of Dry Stuff** | | |
| Raw protein | 21.4 | 16.6 |
| Raw fat | 2.2 | 2.1 |
| Fiber | 5.8 | 5.5 |
| NFE (Nitrogen Free Extract) | 64.8 | 70.6 |
| Ash | 5.8 | 5.2 |
| Feed units per kg dry stuff | 1.18 | 1.16 |
| Digestible protein, g/unit | 154.0 | 118.0 |
| Digestible lysin, g/unit | 8.1 | 5.5 |
| Digestible Threonine, g/unit | 5.7 | 4.2 |

* Content per g: 1500 i.e., A-vitamin, 500, i.e., $D_3$-vitamin, 0.01 mg $B_{12}$-vitamin, 10 mg alpha--tocoferolacetat, 2.5 mg $B_2$-vitamin, 7.5 mg pantothenic acid, 0.66 mg natriumselenit, 50 mg zincoxyde, 62.5 mg copper sulphate, 62.5 mg manganese sulphate, 2.5 mg cobalt sulphate, 62.5 mg iron sulphate and 0.5 mg kaliumiodid.

IB. England Trial

The study was designed to evaluate the growth promotion potential of TriCP as assessed in pigs reared for bacon production. The test diets were offered continuously until the animals reached 100 kg. The pigs

were then slaughtered after a 2-week withdrawal period. Weight gain, food consumption and feed utilization were measured and at termination carcass data were obtained under commercial conditions.

Thirty-two (32) castrated males and thirty-two (32) female pigs (Sus scrofa) of the Large White type, approximately 11 weeks old at the start of treatment, were used in the study. They were obtained from R. Beedles, Shadymoor, Dorrington, Shropshire.

The animals were housed throughout the study in a building designed for bacon pig production in floor pens of concrete construction. Each floor pen incorporated individual metal feeding crates, a communal lying area and a dung passage. The pens also contained automatic drinkers and wheat straw was provided as bedding material. The building was ventilated by means of baffled inlets along the side walls and 2 extract fans in the central roof ridge.

Four feed premixes were used to produce four experimental diets, three containing TriCP at differing inclusion levels and one control diet without TriCP. The feed premixes were not identified until after termination of the study.

The animals were delivered to Huntingdon Research Center (HRC) at approximately 8 weeks of age and were maintained on basal diet for 3 weeks prior to the start of the experimental period. All pigs were inspected by a veterinary surgeon and were routinely treated with an anthelmintic ("Thiprazole", Merck, Sharp and Dohme) and a swine erysipelas vaccine (Wellcome Foundation Limited).

Immediately before the start of the study, all pigs were allotted a temporary identification number and were weighed and re-examined to assess general health and condition. After discarding spare animals, the pigs selected for use in the study were allocated to treatment according to body weight. In practice this was achieved by stratification by weight (males and females separately) followed by systematic allocation to feeder positions in order starting in the first pen. This procedure minimized the variation in size and weight within pens (blocks). The animals were then tagged with permanent identification numbers to keep track of each animal's identity.

The pigs were offered one of 2 pelleted concentrate rations throughout the study. The first ration was a grower or starter ration with a higher nutrient density. The grower ration was used initially and up to 50 kg body weight, and was replaced by a pig finisher diet from 50 kg to termination. The composition and specification of these 2 basal diets is given in Table 3.

Table 3

| Feed Mixture Composition and Contents in England Trial | | | |
|---|---|---|---|
| | | Pig grower ration (< 50 kg body weight) | Pig finisher ration (> 50 kg body weight) |
| Composition (% w/w) | Barley | 40.51 | 40.51 |
| | Wheat | 10.00 | 10.00 |
| | Maize | 15.00 | 15.00 |
| | Extracted soya bean meal | 14.50 | 11.50 |
| | Provimi 66 fish meal | 3.50 | 2.50 |
| | Weatings | 10.00 | 15.00 |
| | Dicalcium phosphate | 0.44 | 0.44 |
| | Limestone flour | 1.05 | 1.05 |
| | Salt | 0.25 | 0.25 |
| | Molasses | 2.50 | 2.50 |
| | Fat premix (50%) | 2.00 | 1.00 |
| | Beta 114* | 0.25 | 0.25 |
| Pellet size (mm diameter) | | 4 | 10 |
| Theoretical analysis (%) | Oil | 3.25 | 2.85 |
| | Crude Protein | 16.95 | 15.66 |
| | Fiber | 4.48 | 4.61 |
| | Total digestible nutrients | 71.81 | 70.93 |
| | Digestible energy MJ/kg (approx.) | 13.00 | 12.75 |
| | Lysine | 0.89 | 0.77 |
| | Methionine and cystine | 0.56 | 0.51 |
| | Calcium | 0.87 | 0.81 |
| | Phosphorus | 0.60 | 0.59 |
| | Salt | 0.47 | 0.46 |

* Mineral/vitamin premix (B.P. Nutrition) - formulated without the standard inclusion of copper at 180 ppm

A restricted feeding regime was used in accordance with normal commercial practice. The pigs were given 2 equal feeds per day, the feed allowance based on body weight as follows:

| Body weight (kg) | Feed allowance (g per feed) |
|---|---|
| ≦ 25.0 | 500 |
| 25.5 to 30.0 | 600 |
| 30.5 to 35.0 | 700 |
| 35.5 to 40.0 | 800 |
| 40.5 to 45.0 | 900 |
| 45.5 to 50.0 | 1000 |
| 50.5 to 55.0 | 1100 |
| 55.5 to 60.0 | 1200 |
| 60.5 to 65.0 | 1300 |
| > 65.0 | 1400 |

Animals were weighed and feed allowances adjusted at weekly intervals.

All feed refusals were measured and recorded and the total weekly food consumption for each pig was derived.

Test diets were mixed and pelleted in batches of 0.5 or 1.0 tonne as follows: 1 tonne batches of basal diet (2 or 4 as appropriate) were first mixed and retained in meal form in 25 kg bags. These were then divided into 4 lots of 0.5 or 1.0 tonne such that each lot consisted of an equal number of bags selected at random from each basal 1 tonne mix. Test diets were then prepared by returning each 0.5 or 1.0 tonne of meal to the mixer together with the appropriate test premix (pre-weighed and supplied by HRC). The mix was then pelleted to produce the finished diet.

Before the start of the study, 4 x 0.5 tonnes of test diets were prepared using the test premixes at the intended inclusion level of 500 g/tonne and samples of the diets taken both before and after pelleting were submitted for assay. All samples were assayed at 40 percent below nominal levels and further test diets were prepared using 750 g of premix/tonne. Analysis of this second batch of diets showed recovery of target levels of TriCP. This was the required premix inclusion level throughout the trial.

In all test diets used throughout the study, premixes were included at a level of 750 g per tonne of finished feed. The levels of test compound in each diet (ppm) after termination of the study, are as shown in Table 1.

On completion of its 2 week withdrawal period (basal diet only) each pig was sent for slaughter at an approved commercial abattoir. The eviscerated carcasses were weighed and graded and this information was returned to HRC.

All pigs were maintained on test diet from the start of the test period until they reached 100 kg live weight. They were then fed with basal diet only for a period of 2 weeks before slaughter. All pigs were observed daily and any clinically abnormal signs were noted.

IC. Germany Trial

In a test on growing pigs, the effect of the additive 3,5,6-trichloro-2-pyridinol (TriCP) on the body weight gain, feed consumption and feed conversion was investigated. The aim of the investigations was to determine the effectiveness of the additive from the nutrition physiology point of view.

A total of 168 pigs consisting of equal numbers of castrated males and females were available for the tests. The pigs were largely of uniform genetic origin. The animals were numbered individually. At the start of the test each pig had a body weight of about 30 kg. Fattening was continued until a final weight of about 105 kg was achieved.

The 168 test pigs were divided into four treatment groups at the start of the test, so that 42 animals were subjected to treatment in each case. Each test group was subdivided into seven sub-groups with six animals in each sub-group. These six pigs were kept together in a pen.

The four test groups were treated as follows. One group was kept as a control group without addition of the additive. Three further groups were used as test groups, in which TriCP was administered in a dosage of 50 ppm, 75 ppm or 100 ppm in the feed.

The composition of the test feed met the requirements of the "Feed Regulations in the Federal Republic of Germany". Table 4 gives details of the nutrient content of the test feed.

Table 4

Contents of the Test Mixtures in Percent in Germany Trial

Preparatory Feeding

| | |
|---|---|
| Raw Protein | 16.50 |
| Lysine | 0.90 |
| Raw Fat | 4.25 |
| Starch | 39.00 |
| Sugar | 4.50 |
| Crude Fiber | 3.75 |
| Calcium | 0.90 |
| Phosphorus | 0.60 |
| Sodium | 0.20 |

Final Fattening

| | |
|---|---|
| Raw Protein | 15.00 |
| Lysine | 0.70 |
| Raw Fat | 4.75 |
| Starch | 38.25 |
| Sugar | 4.70 |
| Crude Fiber | 4.40 |
| Calcium | 0.85 |
| Phosphorus | 0.55 |
| Sodium | 0.18 |

The test animals were kept in a closed house on the experimental far at Göttingen University. The environmental conditions were largely standardized. The feed was distributed twice daily at fixed times (8 a.m. and 3 p.m.). Water consumption was ensured by automatic drinking bowls.

The following criteria were checked during the test. The body weights of the individual animals were weighed every 28 days. This interval was reduced to 21 days for some sub-groups at the end of the test. This was necessary, if the body weight trend with a 28-day interval between the last two weighings would have resulted in excessive final weights.

The feed consumption of the quantity of feed consumed daily was recorded. The feed conversion per kg gain was calculated from the feed consumption and the gain in body weight.

ID. Holland Trial

In this trial the effect of oral administration of three dosages of TriCP on weight gain and feed conversion efficiency is described. The effect of TriCP on these criteria is compared with that of a control group without a growth promotor.

The study was carried out with 160 pigs distributed among four groups of 40 animals each. Each group contained five repetitions of eight animals, (2 pens with 8 castrated males, 3 pens with 8 females). The trial was comprised of two periods. A first period of 0 to 6 weeks, wherein the live weight of the pigs was 22 to 45 kg, in which pigs receiving 50, 75 or 100 ppm TriCP were compared with a control group without supplementation. The second period was 6 to 16/18 weeks, wherein the live weight of the pigs was 45 to 100 kg, in which pigs receiving 50, 75 or 100 ppm TriCP were compared with a control group without supplementation.

The pigs were of the cross Dutch Landrace x Large White. For this experiment 64 castrated males and 96 females of a known offspring were purchased. The animals were selected in such a way that the variation in age and live weight was as small as possible. At the beginning of the feed trial the age of the piglets was eight weeks; the average live weight of the females was 21.5 kg and of the castrated males 21.9 kg.

Before the feed trial commenced the pigs were fed a commercial ration containing an antibiotic. Six

weeks after arrival of the animals at the Institute they were treated with 0.25 g piperazine adipas per kg body weight.

One day after arrival of the animals at the Institute they were distributed among the four treatment groups in such a way, that the groups were comparable with respect to sex, parentage, age and live weight. For both sexes the average initial live weight and their standard deviation was equalized as well as possible for each group. In general each group comprised no more than one animal of the same litter.

The pigs were housed in a piggery of the Danish type with partly slatted floors without straw. The piggery holds 24 pens. Eight animals were housed in each pen. Ten pens held castrated males and 14 pens females. The piggery is lighted, heated and ventilated artificially.

The minimal and maximal temperature in the piggery varied during the whole experimental period between 15° and 22° C.

The experiment consisted of two treatment periods of 6 and 10 weeks, respectively, during which the experimental rations were fed.

The composition of the diets is given in Table 5. The diets for the animals of groups 1 to 4 contained no extra copper.

## Table 5

### Feed Mixture, Composition and Contents in Holland Trial

| Ingredient | 0 - 6 weeks | 6 - 16/18 weeks |
|---|---|---|
| Wheat | 10.0 | 11.0 |
| Barley | 27.0 | - |
| Corn germmeal feed | - | 15.0 |
| Wheat middlings | 5.0 | 20.0 |
| Tapioca | 23.2 | 32.7 |
| Soya oil | 1.5 | 2.5 |
| Whey powder (delactosed) | 2.0 | - |
| Whey powder | 5.0 | - |
| Soybean oilmeal | 23.0 | 15.6 |
| Vitamins, minerals ** | 3.3 | 3.2 |
| **Calculated contents (in %)** | | |
| Crude protein | 17.0(16.6*) | 13.6(14.4*) |
| Lysine | 0.96 | 0.70 |
| Methionine + cystine | 0.55 | 0.45 |
| Net energy (kcal/kg) | (2190) | (2185) |
| Calcium | 0.95(0.96*) | 0.80(0.89*) |
| Phosphorous | 0.75(0.70*) | 0.65(0.65*) |

* Analyzed

** The vitamin premix supplied per 1 kg diet: 6,000 I.U. Vitamin A, 1,200 I.U. Vitamin $D_3$, 25 mg Vitamin E, 2 mg Vitamin K, 4 mg Riboflavin, 20 mg Niacin, 10 mg d-Pantothenic acid, 80 mg Choline chloride, 0.030 mg Vitamin $B_{12}$, 0.54 mg Kl and 4.65 mg $CoSO_4 \cdot 7H_2O$.

After mixing the basal ration, it was divided into five sub-charges and then supplemented respectively

with carrier and 50, 75 and 100 ppm TriCP. The mixing was done in several batches. Sampling of batches followed by analysis of crude protein, calcium and phosphorus were carried out.

The animals were fed in groups of eight, twice a day, according to a fixed scheme based on the mean body weight of the animals of each pen. If the pigs of one or more pens grow faster than those in other pens, they were offered more feed. After each feeding, the meal for the next feeding was mixed with water in a 1:1 ratio. The pigs had free access to fresh water via automatic drinkers.

State of health of the pigs was observed daily. Live weight of the pigs was determined every three weeks. Feed conversion efficiency (kg feed/kg live weight gain) was calculated per pen of eight animals at the time of each weighing. The animals were slaughtered 16, 17 or 18 weeks after the start of the experiment. The experimental rations were fed till 24 hours before slaughter, except for some castrated male animals of group 4 (75 ppm): for one animal of this group the withdrawal time was 0 days, for three animals three days and for three animals seven days.

Summary of Results for European Trials

After the end of the finisher diets, individual pig weights and amount of feed consumed were obtained in order to determine weight gains and efficiency of feed use. The results for swine average daily weight gain for pigs receiving TriCP are presented in Table 6. The results for the feed conversion efficiency by the pigs receiving TriCP are presented in Table 7.

Table 6

| SWINE AVERAGE DAILY WEIGHT GAIN (ADG) IN GRAMS/DAY DURING FINISHING PERIOD (50 TO 100 KG) | | | | | |
|---|---|---|---|---|---|
| Country | Number of Pigs | Concentration of TriCP in Feed in ppm | | | |
| | | 0 | 50 | 75 | 100 |
| Denmark | 128 | 696(0)τ | 730(+34)τ | 712(+16) | 729(+33) |
| England | 64 | 777(0) | 765(-12) | 759(-18) | 820(+43) |
| Germany | 168 | 705(0) | 792(+87) | 732(+27) | 697(-8) |
| Holland | 160 | 710(0) | 747(+37) | 738(+28) | 738(+28) |

τ Numbers in parenthesis ( ) represent the difference in weight gain between those animals receiving TriCP in their diets and those receiving "0" or no TriCP.

As can be seen in Table 6, TriCP was effective in increasing the daily weight gain in swine receiving feed containing from 50 to 100 ppm TriCP.

Table 7

| FEED CONVERSION EFFICIENCY BY PIGS RECEIVING TRICP | | |
|---|---|---|
| Country | Concentration of TriCP in feed (ppm) | Feed Efficiency of pigs 50-100 kg (%) |
| England | 0 | 100.0 |
| | 50 | 101.8 |
| | 75 | 104.0 |
| | 100 | 93.3 |
| Germany | 0 | 100.0[1] |
| | 50 | 89.4[1] |
| | 75 | 94.7[1] |
| | 100 | 101.1[1] |

[1] Measurements taken at pig weights 65 to 100 kg

As can be seen in Table 7, pigs receiving TriCP achieved a feed conversion efficiency of 6 percent or more

over the pigs not receiving TriCP, where average daily weight gains were also increased as in Table 6.

II. Protocol for United States Trials

Example 6: The anabolic effect of sodium 3,5,6-trichloro-2-pyridinate as exhibited by an increase in average daily gain and an increase in feed conversion efficiency for swine.

Three-hundred-forty (340) swine averaging 20 kg each, were allotted into 5 treatments of 6 replicates of 8 animals and 2 replicates of 10 animals. The animals were fed a two part basal diet consisting of:

| | % of Diet | |
|---|---|---|
| Ingredient | Grower Diet | Finisher Diet |
| Ground Corn | 79.60 (722 kg/ton) | 84.64 (767 kg/ton) |
| Soybean Oil Meal | 17.85 (162 kg/ton) | 12.91 (117 kg/ton) |
| Defluorinated Rock Phosphate | 1.80 (16 kg/ton) | 1.80 (16 kg/ton) |
| Salt | 0.50 (4.5 kg/ton) | 0.50 (4.5 kg/ton) |
| Trace Mineral & Vitamin Premix | 0.25 (2.2 kg/ton) | 0.25 (2.2 kg/ton) |

whereby the grower diet was fed to animals until they reached a weight of 55 kg and the finisher diet was fed to animals thereafter.

To these diets, sodium 3,5,6-trichloro-2-pyridinate, hereinafter referred to as Na-TriCP, was added at a rate so that the different treatment groups received 1 mg/kg/day, 2 mg/kg/day, 3 mg/kg/day and 4 mg/kg/day of Na-TriCP in accordance with the invention. The control group was fed the basal diet alone with no addition of Na-TriCP. After a 112 day total feeding period, weight gains and feed consumption of the animals were determined. The results obtained are tabulated as follows:

| | Average Dose of Sodium 3,5,6-Trichloro-2-pyridinate, mg/kg/day* | | | | |
|---|---|---|---|---|---|
| Treatment | 0 | 1 | 2 | 3 | 4 |
| Avg. Daily Feed Consumption, lb. | 5.32 | 5.32 | 5.43 | 5.68 | 5.59 |
| Avg. Daily Gain, lb. | 1.54 | 1.61 | 1.63 | 1.72 | 1.72 |
| Feed Conversion Efficiency | 3.46 | 3.32 | 3.34 | 3.31 | 3.26 |

*The average daily dose of sodium 3,5,6-trichloropyridinate in mg/kg was achieved by incorporating Na-TriCP into the feed in 25 ppm, 50 ppm, 75 ppm and 100 ppm which correspond to an average dose of 1 mg/kg/day, 2 mg/kg/day, 3 mg/kg/day and 4 mg/kg/day respectively. This is based on the average daily feed intake and the average weight of the animals. The actual dose of Na-TriCP may have varied from day to day.

Example 7: The anabolic effect of 3,5,6-trichloro-2-pyridinol as exhibited by an increase in milk production of lactating cows.

Six (6) lactating Holstein-Friesian cows, in their 4th to 10th week of lactation, were allotted into 2 treatments consisting of 3 cows each. All animals were fed the same ration except the second group which was fed the ration plus 3,5,6-trichloro-2-pyridinol, hereinafter referred to as TriCP, at levels that were increased at 14 day intervals for 6 feeding periods. At the end of these feeding periods, a 14 day withdrawal period was instituted. The concentrations of TriCP added to the ration were as follows:

| Feeding Period (14 days) | Concentration of TriCP in ppm | |
|---|---|---|
| | Group 1 (Control) | Group 2 |
| 1 (prefeeding) | 0 | 0 |
| 2 | 0 | 1 |
| 3 | 0 | 3 |
| 4 | 0 | 10 |
| 5 | 0 | 30 |
| 6 | 0 | 100 |
| 7 | 0 | 0 (withdrawal) |

Milking and feeding were done on a morning and evening schedule for each cow. The feeding was done after the milking.

The average daily feed consumption in kg; the average daily dry matter milk production in kg; and the milk production efficiency are as follows:

| | Group 1 (Control) | Group 2 |
|---|---|---|
| Avg. Daily Feed Consumption in kg | 18.52 | 18.18 |
| Avg. Daily Dry Matter Milk Production per day in kg* | 2.15 | 2.41 |
| Milk production Efficiency | 8.61 | 7.54 |

*the dry matter milk production is equal to 12% of the actual milk production.

Example 8: The anabolic effect of sodium 3,5,6-trichloro-2-pyridinate as exhibited by an increase in average daily gain and an increase in feed conversion efficiency for beef cattle.

Two-hundred fifty-six (256) steer calves averaging 283 kg (625 lbs) were allotted into four (4) treatments of 8 replicates of 8 animals, i.e., 64 animals per treatment. The animals were assigned to replicates in a randomized complete block design. The animals were fed a two part basal diet consisting of:

| | % of Diet (by Weight) | |
|---|---|---|
| Ingredient | Intermediate Ration | Finishing Ration |
| Dehydrated Alfalfa Meal | 10.0 | 5.0 |
| Cottonseed Hulls | 15.0 | 10.0 |
| Steam Rolled Corn | 58.0 | 74.8 |
| Soybean Meal (44%) | 10.0 | 3.0 |
| Calcium Carbonate | 1.0 | 0.7 |
| Na Tripolyphosphate | 0.5 | 0.3 |
| Cane Molasses | 5.0 | 5.0 |
| Trace Mineralized Salt | 0.5 | 0.5 |
| Urea | None | 0.7 |

whereby the intermediate ration was fed to animals the first 28 days of the experiment and the finishing ration was fed to animals thereafter. All diets and water were available to the animals on an ad libitum basis.

To the above diets, sodium 3,5,6-trichloro-2-pyridinate (Na-TriCP) was added in amounts sufficient to satisfy the dose requirements of Na-TriCP each treatment was to receive, namely:

| Treatment | Concentration of Na-TriCP in Diet (wt. percent) | |
|---|---|---|
| 1 | 0 | (Control) |
| 2 | 0.005 | (50 ppm) |
| 3 | 0.0075 | (75 ppm) |
| 4 | 0.01 | (100 ppm) |

The control diet contained no Na-TriCP while the other three treatments were fed diets containing Na-TriCP in concentrations listed above. After a 112 day total feeding period, weight gains and feed conversion efficiency improvements were determined. The average initial weight, average 112-day weight, average daily gain, average feed intake per day and, average feed conversion efficiency (feed consumed/body weight gain) are tabulated as follows:

| | Treatment | | | |
|---|---|---|---|---|
| | 1 | 2 | 3 | 4 |
| Average initial weight (kg) | 285 | 285 | 284 | 285 |
| Average 112-day weight (kg) | 424 | 433 | 432 | 429 |
| Average daily gain (kg) | 1.24 | 1.32 | 1.30 | 1.28 |
| Average feed intake/day | 9.40 | 9.50 | 9.40 | 9.40 |
| Average feed conversion efficiency | 7.52 | 7.23 | 7.14 | 7.39 |

All animal weights were calculated after a 16 hour shrink off feed and water. The incorporation of Na-TriCP into the feed in 50 ppm, 75 ppm and 100 ppm corresponds approximately to an average daily dose of 1.55 mg/kg of body weight/day, 2.33 mg/kg of body weight/day and 3.1 mg/kg of body weight/day respectively. This is based on the average daily feed intake and the average weight of the animals. The actual individual doses of Na-TriCP may have varied from day to day.

## Claims

1. A method of producing an anabolic response in animals selected from the group of swine, cattle, sheep or poultry comprising administering to the animal an anabolically effective amount of one or a mixture of two or more active compounds of the following formula

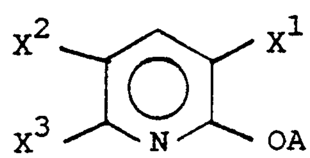

(I)

wherein

$X^1$, $X^2$ and $X^3$ independently represent halogen, optionally chloro;

A represents hydrogen, alkali metal, alkaline earth metal, zinc, iron, manganese or

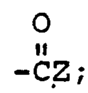 and

Z represents $C_1$-$C_{11}$ alkyl, $C_1$-$C_{11}$ haloalkyl or $C_1$-$C_{11}$ hydroxyalkyl (methods for treatment of the animal body by surgery or therapy and diagnostic methods practised on the animal body being excluded).

2. The method of Claim 1 wherein said compound is 3,5,6-trichloro-2-pyridinol, or the sodium salt thereof.

3. The method of Claim 1 wherein said anabolic response is exhibited by an increase in average daily weight gain, feed conversion efficiency or by an increase in milk production in a lactating animal.

4. The method of Claim 1 wherein said active compound is administered to swine at a rate of from 0.01 to 20 milligrams per kilogram of body weight per day.

5. The method of Claim 1 wherein the active ingredient is administered to swine at a rate of 50 to 100 grams/ton in the animal feed.

6. An animal feed for swine, cattle, sheep or poultry comprising from 0.00005 to 15 percent by weight of one or a mixture of two or more anabolically active compounds of the formula

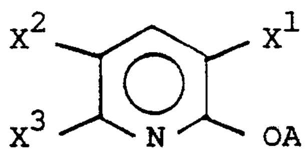

(I)

wherein

$X^1$, $X^2$ and $X^3$ independently represent halogen, preferably chloro;

A represents hydrogen, alkali metal, alkaline earth metal, zinc, iron, manganese or

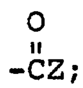

and

Z represents $C_1$-$C_{11}$ alkyl, $C_1$-$C_{11}$ haloalkyl or $C_1$-$C_{11}$ hydroxyalkyl.

7. The animal feed of Claim 6 which when fed to an animal provides to the animal from 0.01 to 20 milligrams per kilogram of body weight per day of one or more of said active compounds.

8. The animal feed of Claim 6 which is a swine feed wherein the active compound is present in an amount of from 0.5 to 1600 grams per ton of finished feed (Dry Matter Basis).

9. The animal feed of Claims 6, 7 and 8 wherein said active compound is 3,5,6-trichloro-2-pyridinol or the sodium salt thereof.

10. The animal feed of Claim 6 wherein said active compound is present in an amount of from 0.0004 to 0.18 percent by weight.

11. The animal feed of Claim 6 which is a swine feed wherein the active compound is present in an amount of from 50 to 100 grams per ton of finished feed (Dry Matter Basis).

**Revendications**

1. Procédé pour produire une réponse anabolique chez des animaux choisis parmi les porcins, les bovins, les ovins ou la volaille, qui comprend l'administration à l'animal d'une quantité, stimulant l'anabolisme; d'un composé actif, ou d'un mélange de deux ou plusieurs composés actifs, de formule suivante

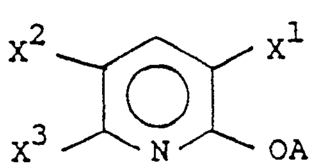

(I)

dans laquelle

$X^1$, $X^2$ et $X^3$ représentent, indépendamment l'un de l'autre, un atome d'halogène, de préférence de chlore,

A représente un atome d'hydrogène, de métal alcalin, de métal alcalino-terreux, de zinc, de fer, de manganèse, ou -C(=O)Z, et

Z représente un goupe alkyle en $C_{1-11}$, halogénoalkyle en $C_{1-11}$ ou hydroxyalkyle en $C_{1-11}$,

(à l'exclusion des procédés de traitement du corps d'un animal par chirurgie ou-thérapeutique et des méthodes de diagnostic pratiquées sur le corps d'un animal).

2. Procédé selon la revendication 1, dans lequel ledit composé est le 3,5,6-trichloro-2-pyridinol ou son sel de sodium.

3. Procédé selon la revendication 1, dans lequel ladite réponse anabolique se manifeste par une augmentation du gain de poids journalier moyen, du rendement de conversion des aliments ou par une augmentation de la production de lait chez un animal donneur de lait.

4. Procédé selon la revendication 1, dans lequel ledit composé actif est administré à des porcs à raison de 0,01 à 20 mg par kg de poids corporel par jour.

5. Procédé selon la revendication 1, dans lequel ledit ingrédient actif est administré à des porcs à raison de 50 à 100 g par tonne dans l'alimentation animale.

6. Alimentation animale pour porcs, bovins, ovins ou volaille, comprenant de 0,00005 à 15 % en poids d'un composé actif comme anabolisant, ou d'un mélange de deux ou plusieurs composés actifs comme anabolisants, de formule

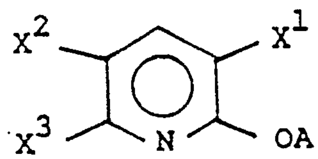

(I)

dans laquelle

$X^1$, $X^2$ et $X^3$ représentent, indépendamment l'un de l'autre, un atome d'halogène, de préférence de chlore,

A représente un atome d'hydrogène, de métal alcalin, de métal alcalino-terreux, de zinc, de fer, de manganèse, ou -C(=O)Z, et

Z représente un goupe alkyle en $C_{1-11}$, halogénoalkyle en $C_{1-11}$ ou hydroxyalkyle en $C_{1-11}$.

7. Alimentation animale selon la revendication 6, qui, lorsqu'elle est administrée à un animal, lui apporte de 0,01 à 20 mg par kg de poids corporel, par jour, d'un ou plus d'un desdits composés actifs.

8. Alimentation animale selon la revendication 6, qui est une alimentation pour porcs dans laquelle le composé actif est présent à raison de 0,5 à 1600 g par tonne d'aliment fini (produit sec).

9. Alimentation animale selon les revendications 6, 7 et 8, dans laquelle ledit composé actif est le 3,5,6-trichloro-2-pyridinol ou son sel de sodium.

10. Alimentation animale selon la revendication 6, dans laquelle ledit composé actif est présent à raison de 0,0004 à 0,18 % en poids.

11. Alimentation animale selon la revendication 6, qui est une alimentation pour porcs dans laquelle le composé actif est présent à raison de 50 à 100 g par tonne d'aliment fini (produit sec).

**Patentansprüche**

1. Verfahren zur Erzeugung einer anabolischen Reaktion in Tieren, ausgewählt aus der Gruppe von

Schweinen, Rindern, Schafen und Geflügel, umfassend das Verabreichen dem Tier einer anabolisch wirksamen Menge an einer oder einem Gemisch von zwei oder mehreren aktiven Verbindungen der folgenden Formel

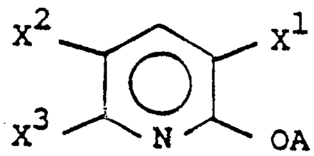

(I)

worin
$X^1$, $X^2$ und $X^3$ unabhängig Halogen, fakultativ Chlor darstellen,
A Wasserstoff, Alkalimetall, Erdalkalimetall, Zink, Eisen, Mangan oder

$-\overset{\mathrm{O}}{\overset{\|}{\mathrm{C}}}\mathrm{Z}$ darstellt und

Z $C_1$-$C_{11}$ Alkyl, $C_1$-$C_{11}$ Haloalkyl oder $C_1$-$C_{11}$ Hydroxyalkyl darstellt (wobei Verfahren zur Behandlung des Tierkörpers durch Operation oder Therapie und diagnostische Methoden, die am Tierkörper durchgeführt werden, ausgeschlossen sind).

2. Verfahren nach Anspruch 1, worin die Verbindung 3,5,6-Trichlor-2-pyridinol oder das Natriumsalz davon ist.

3. Verfahren nach Anspruch 1, worin sich die anabolische Reaktion durch einen Anstieg der mittleren täglichen Gewichtszunahme, der Futterunwandlungs-Wirksamkeit oder durch ein Anwachsen der Milchproduktion in einem laktierenden Tier ausdrückt.

4. Verfahren nach Anspruch 1, worin die aktive Verbindung Schweinen mit einer Rate von 0,01 bis 20 Milligramm pro Kilogramm Körpergewicht pro Tag verabreicht wird.

5. Verfahren nach Anspruch 1, worin die aktive Verbindung Schweinen mir einer Rate von 50 bis 100 Gramm pro Tonne im Tierfutter verabreicht wird.

6. Tierfutter für Schweine, Rinder, Schafe oder Geflügel, enthaltend von 0,00005 bis 15 Gew.-% an einer oder einem Gemisch von zwei oder mehreren anabolisch aktiven Verbindungen der folgenden Formel

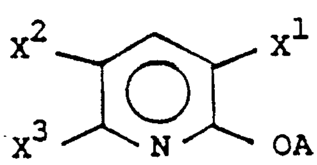

(I)

worin
$X^1$, $X^2$ und $X^3$ Unabhängig Halogen, vorzugsweise Chlor darstellen,
A Wasserstoff, Alkalimetall, Erdalalkalimetall, Zink, Eisen, Mangan oder

$-\overset{\mathrm{O}}{\overset{\|}{\mathrm{C}}}\mathrm{Z}$ darstellt und

Z $C_1$-$C_{11}$ Alkyl, $C_1$-$C_{11}$ Haloalkyl oder $C_1$-$C_{11}$ Hydroxyalkyl darstellt.

7. Tierfutter nach Anspruch 6, das, wenn es einem Tier verfüttert wird, dem Tier von 0,01 bis 20 Milligramm pro Kilogramm Körpergewicht pro Tag an einer oder mehreren der aktiven Verbindungen liefert.

8. Tierfutter nach Anspruch 6, das ein Schweinefutter ist, worin die aktive Verbindung in einer Menge von 0,5 bis 1600 Gramm pro Tonne Fertigfutter (auf Basis der Trockensubstanz) vorhanden ist.

9. Tierfutter nach den Ansprüchen 6, 7 und 8, worin die aktive Verbindung 3,5,6-Trichlor-2-pyridinol oder das Natriumsalz davon ist.

10. Tierfutter nach Anspruch 6, worin die aktive Verbindung in einer Menge von 0,0004 bis 0,18 Gew.-% vorhanden ist.

11. Tierfutter nach Anspruch 6, das ein Schweinefutter ist, worin die aktive Verbindung in einer Menge von 50 bis 100 Gramm pro Tonne Fertigfutter (auf Basis der Trockensubstanz) vorhanden ist.